(19) **Europäisches Patentamt / European Patent Office / Office européen des brevets**

(11) **EP 4 126 105 B1**

(12) **FASCICULE DE BREVET EUROPEEN**

(45) Date de publication et mention de la délivrance du brevet:
**15.04.2026 Bulletin 2026/16**

(21) Numéro de dépôt: **21713699.3**

(22) Date de dépôt: **25.03.2021**

(51) Classification Internationale des Brevets (IPC):
***A61M 1/36*** (2006.01)

(52) Classification Coopérative des Brevets (CPC):
**A61M 1/3687; A61M 1/3486; A61M 1/3679;**
A61M 2202/0417

(86) Numéro de dépôt international:
**PCT/EP2021/057849**

(87) Numéro de publication internationale:
**WO 2021/191396 (30.09.2021 Gazette 2021/39)**

(54) **COLONNE D'APHÉRÈSE POUR LE TRAITEMENT DE LA POLYARTHRITE RHUMATOÏDE**

APHERESESÄULE ZUR BEHANDLUNG VON RHEUMATOIDER POLYARTHRITIS

APHERESIS COLUMN FOR TREATING RHEUMATOID POLYARTHRITIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.03.2020 FR 2002992**

(43) Date de publication de la demande:
**08.02.2023 Bulletin 2023/06**

(73) Titulaires:
- **Université Paul Sabatier Toulouse III**
  **31400 Toulouse (FR)**
- **Centre Hospitalier Universitaire de Toulouse**
  **31300 Toulouse (FR)**
- **Institut National de la Santé et de la Recherche Médicale**
  **75013 Paris (FR)**
- **Assistance Publique Hôpitaux de Marseille**
  **13005 Marseille (FR)**
- **Université d'Aix Marseille**
  **13007 Marseille 7 (FR)**

(72) Inventeurs:
- **SERRE, Guy**
  **31059 Toulouse Cedex 9 (FR)**
- **OFFER, Géraldine**
  **31059 Toulouse Cedex 9 (FR)**
- **CHABOD, Marianne**
  **31059 Toulouse Cedex 9 (FR)**
- **PANCARTE, Mikael**
  **31059 Toulouse Cedex 9 (FR)**
- **ROUDIER, Jean**
  **13009 Marseille (FR)**
- **BALANDRAUD, Nathalie**
  **13009 Marseille (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
WO-A1-2006/048556     FR-A1- 2 908 134

**Description**

**Domaine technique de l'invention**

La présente invention concerne une colonne d'aphérèse ayant un support solide comprenant une composition de peptides citrullinés et ladite composition immobilisée dans la colonne d'aphérèse pour son utilisation dans une méthode de traitement d'une maladie auto-immune à auto-anticorps anti-protéines citrullinées.

**Arrière-plan technique**

La polyarthrite rhumatoïde (PR) est la plus fréquente maladie auto-immune humaine mais aussi le plus fréquent rhumatisme inflammatoire chronique. Elle affecte 0,5 à 1% de la population des pays développés et est caractérisée par une inflammation chronique et destructrice des articulations. Elle s'accompagne de la production d'anticorps antiimmunoglobulines, appelés « facteurs rhumatoïdes » ainsi que d'anticorps anti-peptides/protéines citrullinés (AAPC). Les AAPC apparaissent souvent des années avant le début de la maladie (Rantapää-Dahlqvist S, et al. Arthritis Rheum. 2003 Oct;48(10):2741-9 ; Nielen MM, et al. Arthritis Rheum. 2004 Feb;50(2):380-6) et sont présents dans 70 à 80% des PR établies (De Rycke L, et al. Ann. Rheum. Dis. 2004 63:12, 1587-1593, 2004). Du fait de leur grande spécificité et de leur présence au début de la maladie, ils ont une grande valeur diagnostique. Souvent associés à une évolution plus sévère, ils ont une relative valeur pronostique. Enfin, ils ont également une valeur prédictive, puisqu'ils peuvent prédire l'évolution d'une arthrite indifférenciée vers une PR. Les AAPC peuvent être détectés grâce à différents tests ELISA (Enzyme-Linked ImmunoSorbent Assay : test immunoenzymatique sur plaque de microtitration). Les tests classiquement utilisés en pratique médicale sont des tests commerciaux dits anti-CCP (anti-Cyclic citrullinated peptides). Le test développé et utilisé par les inventeurs est le test AhFibA (Anti-human Fibrinogen Antibodies) qui détecte les anticorps reconnaissant le fibrinogène citrulliné. Les résultats des tests AhFibA et anti-CCP sont largement recouvrants mais cependant discordants chez 5 à 10% des patients.

Dès que la maladie est diagnostiquée, afin de réduire le risque d'évolution vers des lésions articulaires irréversibles, sont prescrits divers traitements plus ou moins spécifiques. Après les corticoïdes, les médicaments immunosuppresseurs sont devenus le traitement de première intention prescrit à la majorité des patients. Dans les formes résistantes à ces traitements sont instituées, parfois successivement, diverses biothérapies de type anti-TNF-alpha, anti-IL6R, anti-CD20. Tous ces traitements doivent être poursuivis de manière chronique, ne sont pas toujours efficaces ou ne le sont que pendant un temps limité et, de plus, présentent tous des effets secondaires plus ou moins graves. Enfin, chez certains patients, malgré la mise en œuvre de cet arsenal thérapeutique, la maladie résiste et progresse.

Il demeure donc nécessaire de trouver de nouveaux moyens de traiter la PR, qui soient toujours efficaces et n'entraînent pas d'effets secondaires invalidants pour le patient.

Ces dernières années, le rôle des AAPC dans le déclenchement et l'entretien de l'arthrite, par formation de complexes immuns avec le fibrinogène citrulliné présent dans les articulations des patients, démontré par les inventeurs, a été largement reconnu par la communauté internationale. Ces complexes immuns induisent en effet, via divers mécanismes effecteurs, la sécrétion de cytokines pro-inflammatoires, notamment du TNF-alpha, qui provoquent et entretiennent l'inflammation articulaire et la synthèse des AAPC. FR2908134 divulgue des peptides citrullinés et leur utilisation comme médicament pour le traitement de la poly arthrite rhumatoïde.

**Résumé de l'invention**

Les inventeurs ont identifié à partir de la fibrine humaine citrullinée, des peptides citrullinés, dits immunodominants, qui sont les cibles préférentielles des AAPC et donc capables de se lier à eux spécifiquement. Les inventeurs ont eu l'idée de débarrasser l'organisme des patients des AAPC pathogènes à l'aide de ces peptides. Les inventeurs ont ainsi trouvé qu'en utilisant une colonne dans laquelle sont immobilisés des peptides citrullinés donnés, ils obtenaient des taux d'épuration en AAPC de plasma de patients proches de 100%. Ils ont alors conçu un nouveau dispositif pour le traitement des maladies auto-immunes à auto-anticorps anti-protéines citrullinées telles que la polyarthrite rhumatoïde.

Un objet de la présente invention concerne par conséquent une colonne d'aphérèse chargée d'un support solide comprenant une composition qui comprend au moins un peptide choisi dans le groupe consistant en :

- le peptide, dit $\alpha 171\text{-}185_{Cit}$, ayant la séquence d'acides aminés VDIDIKIX$_1$SCX$_2$GSCS (SEQ ID NO : 8) où X$_1$ et X$_2$ représentent chacun un résidu citrullyl,
- le peptide, dit $\alpha 621\text{-}635_{Cit}$, ayant la séquence d'acides aminés X$_1$GHAKSX$_2$PVX$_3$GIHTS (SEQ ID NO : 12) où X$_1$, X$_2$ et X$_3$ représentent chacun un résidu citrullyl,
- le peptide, dit $\beta 60\text{-}74_{Cit\text{-}NH2}$, ayant la séquence d'acides aminés X$_1$PAPPPISGGGYX$_2$AX$_3$ (SEQ ID NO: 15) où X$_1$ et

$X_2$ représentent chacun un résidu citrullyl et $X_3$ représente un dérivé citrullyl avec un groupement carboxamide à la place du groupement carboxyle et

- le peptide, dit $_{Ac}$-α36-50$_{Cit}$, ayant la séquence d'acides aminés GPX$_1$VVEX$_2$HQSACKDS (SEQ ID NO : 6) où le résidu G en N-terminal est acétylé et où $X_1$ et $X_2$ représentent chacun un résidu citrullyl et/ou le peptide, dit α36-50$_{Cit}$, ayant la séquence d'acides aminés GPX$_1$VVEX$_2$HQSACKDS (SEQ ID NO : 5) où $X_1$ et $X_2$ représentent chacun un résidu citrullyl,

dont le ou les peptides sont immobilisés directement ou indirectement sur le support.

La présente invention concerne également une colonne d'aphérèse chargée d'un support solide comprenant une composition qui comprend au moins deux peptides choisis dans le groupe consistant en :

- le peptide, dit α171-185$_{Cit}$, ayant la séquence d'acides aminés VDIDIKIX$_1$SCX$_2$GSCS (SEQ ID NO : 8) où $X_1$ et $X_2$ représentent chacun un résidu citrullyl,
- le peptide, dit α621-635$_{Cit}$, ayant la séquence d'acides aminés X$_1$GHAKSX$_2$PVX$_3$GIHTS (SEQ ID NO : 12) où $X_1$, $X_2$ et $X_3$ représentent chacun un résidu citrullyl,
- le peptide, dit β60-74$_{Cit-NH2}$, ayant la séquence d'acides aminés X$_1$PAPPPISGGGYX$_2$AX$_3$ (SEQ ID NO: 15) où $X_1$ et $X_2$ représentent chacun un résidu citrullyl et $X_3$ représente un dérivé citrullyl avec un groupement carboxamide à la place du groupement carboxyle et
- le peptide, dit $_{Ac}$-α36-50$_{Cit}$, ayant la séquence d'acides aminés GPX$_1$VVEX$_2$HQSACKDS (SEQ ID NO : 6) où le résidu G en N-terminal est acétylé et où $X_1$ et $X_2$ représentent chacun un résidu citrullyl et/ou le peptide, dit α36-50$_{Cit}$, ayant la séquence d'acides aminés GPX$_1$VVEX$_2$HQSACKDS (SEQ ID NO : 5) où $X_1$ et $X_2$ représentent chacun un résidu citrullyl.

La présente invention concerne aussi l'utilisation d'une composition comprenant au moins un peptide citrulliné choisi dans le groupe consistant en les peptides ayant des séquences SEQ ID NO : 8, SEQ ID NO : 12, SEQ ID NO : 15, SEQ ID NO : 6 et SEQ ID NO : 5 dans la fabrication d'une colonne d'aphérèse, préférablement une colonne d'aphérèse destinée au traitement d'une maladie auto-immune à auto-anticorps anti-protéines citrullinées telle que la polyarthrite rhumatoïde.

La présente invention concerne également une composition comprenant au moins un peptide citrulliné choisi dans le groupe consistant en les peptides ayant des séquences SEQ ID NO : 8, SEQ ID NO : 12, SEQ ID NO : 15, SEQ ID NO : 6 et SEQ ID NO : 5 pour son utilisation d'une maladie auto-immune à auto-anticorps anti-protéines citrullinées et notamment de la polyarthrite rhumatoïde, où le ou les peptides de la composition sont immobilisés sur un support solide chargé dans une colonne d'aphérèse.

## Description détaillée de l'invention

<u>Composition</u>

La présente invention concerne une colonne d'aphérèse chargée d'un support solide qui comprend une composition comprenant au moins un peptide choisi dans le groupe consistant en :

- le peptide, dit α171-185$_{Cit}$, ayant la séquence d'acides aminés VDIDIKIX$_1$SCX$_2$GSCS (SEQ ID NO : 8) où $X_1$ et $X_2$ représentent chacun un résidu citrullyl,
- le peptide, dit α621-635$_{Cit}$, ayant la séquence d'acides aminés X$_1$GHAKSX$_2$PVX$_3$GIHTS (SEQ ID NO : 12) où $X_1$, $X_2$ et $X_3$ représentent chacun un résidu citrullyl
- le peptide, dit β60-74$_{Cit-NH2}$, ayant la séquence d'acides aminés X$_1$PAPPPISGGGYX$_2$AX$_3$ (SEQ ID NO: 15) où $X_1$ et $X_2$ représentent chacun un résidu citrullyl et $X_3$ représente un dérivé citrullyl avec un groupement carboxamide à la place du groupement carboxyle et
- le peptide, dit $_{Ac}$-α36-50Cit, ayant la séquence d'acides aminés GPX$_1$VVEX$_2$HQSACKDS (SEQ ID NO : 6) où le résidu G en N-terminal est acétylé et où $X_1$ et $X_2$ représentent chacun un résidu citrullyl et/ou le peptide, dit α36-50$_{Cit}$, ayant la séquence d'acides aminés GPX$_1$VVEX$_2$HQSACKDS (SEQ ID NO : 5) où $X_1$ et $X_2$ représentent chacun un résidu citrullyl.

Les différentes séquences sont résumées dans le tableau 1 ci-dessous.

**[Tableau 1]**

| Protéine/peptide | Séquence d'acides aminés | SEQ ID NO |
|---|---|---|
| Chaine α du fibrinogène humain (NP_068657) (AAI01936) | MFSMRIVCLVLSVVGTAWTADSGEGDFLAEGGGVRGPRVVERHQSACKDSDWPFCSDEDWNYK CPSGCRMKGLIDEVNQDFTNRINKLKNSLFEYQKNNKDSHSLTTNIMEILRGDFSSANNRDNTYNR VSEDLRSRIEVLKRKVIEKVQHIQLLQKNVRAQLVDMKRLEVDIDIKIRSCRGSCSRALAREVDLKDY EDQQKQLEQVIAKDLLPSRDRQHLPLIKMKPVPDLVPGNFKSQLQKVPPEWKALTDMPQMRMELE RPGGNEITRGGSTSYGTGSETESPRNPSSAGSWNSGSSGPGSTGNRNPGSSGTGGTATWKPGS SGPGSTGSWNSGSSGTGSTGNQNPGSPRPGSTGTWNPGSSERGSAGHWTSESSVSGSTGQW HSESGSFRPDSPGSGNARPNNPDWGTFEEVSGNVSPGTRREYHTEKLVTSKGDKELRTGKEKVT SGSTTTTRRSCSKTVTKTVIGPDGHKEVTKEVVTSEDGSDCPEAMDLGTLSGIGTLDGFRHRHPDE AAFFDTASTGKTFPGFFSPMLGEFVSETESRGSESGIFTNTKESSSHHPGIAEFPSRGKSSSYSKQ FTSSTSYNRGDSTFESKSYKMADEAGSEADHEGTHSTKRGHAKSRPVRGIHTSPLGKPSLSP | 1 |
| Chaine β du fibrinogène humain (AAA18024) | MKRMVSWSFHKLKTMKHLLLLLLLCVFLVKSQGVNDNEEGFFSARGHRPLDKKREEAPSLRPAPPP ISGGGYRARPAKAAATQKKVERKAPDAGGCLHADPDLGVLCPTGCQLQEALLQQERPIRNSVDEL NNNVEAVSQTSSSSFQYMYLLKDLWQKRQKQVKDNENVVNEYSSELEKHQLYIDETVNSNIPTNL RVLRSILENLRSKIQKLESDVSAQMEYCRTPCTVSCNIPVVSGKECEEIIRKGGETSEMYLIQPDSSV KPYRVYCDMNTENGGWTVIQNRQDGSVDFGRKWDPYKQGFGNVATNTDGKNYCGLPGEYWLG NDKISQLTRMGPTELLIEMEDWKGDKVKAHYGGFTVQNEANKYQISVNKYRGTAGNALMDGASQL MGENRTMTIHNGMFFSTYDRDNDGWLTSDPRKQCSKEDGGGWWYNRCHAANPNGRYYWGGQ YTWDMAKHGTDDGVVWMNWKGSWYSMRKMSMKIRPFFPQQ | 2 |
| α36-50 | GPRVVERHQSACKDS | 3 |
| Ac-α36-50 | GPRVVERHQSACKDS où G en N-terminal est acétylée | 4 |
| α36-50Cit | GPX$_1$VVEX$_2$HQSACKDS où X$_1$ et X$_2$ représentent chacun un résidu citrullyl | 5 |
| Ac-α36-50Cit | GPX$_1$VVEX$_2$HQSACKDS où G en N-terminal est acétylée et où X$_1$ et X$_2$ représentent chacun un résidu citrullyl, | 6 |

EP 4 126 105 B1

| Protéine/peptide | Séquence d'acides aminés | SEQ ID NO |
|---|---|---|
| $\alpha$171-185 | VDIDIKIRSCRGSCS | 7 |
| $\alpha$171-185$_{Cit}$ | VDIDIKIX$_1$SCX$_2$GSCS où X$_1$ et X$_2$ représentent chacun un résidu citrullyl | 8 |
| $\alpha$501-515 | SGIGTLDGFRHRHPD | 9 |
| $\alpha$501-515$_{Cit}$ | SGIGTLDGFX$_1$HX$_2$HPD où X$_1$ et X$_2$ représentent chacun un résidu citrullyl | 10 |
| $\alpha$621-635 | RGHAKSRPVRGIHTS | 11 |
| $\alpha$621-635$_{Cit}$ | X$_1$GHAKSX$_2$PVX$_3$GIHTS où X$_1$, X$_2$ et X$_3$ représentent chacun un résidu citrullyl | 12 |
| $\beta$60-74 | RPAPPPISGGGYRAR | 13 |
| $\beta$60-74$_{Cit}$ | X$_1$PAPPPISGGGYX$_2$AX$_3$ où X$_1$, X$_2$ et X$_3$ représentent chacun un résidu citrullyl | 14 |
| $\beta$60-74$_{Cit-NH2}$ | X$_1$PAPPPISGGGYX$_2$AX$_3$ où X$_1$ et X$_2$ représentent chacun un résidu citrullyl et X$_3$ représente un dérivé citrullyl avec un groupement carboxamide (CONH$_2$) à la place du groupement carboxyle (COOH) terminal. | 15 |

La composition englobe aussi les dérivés ou fragments des peptides définis ci-dessus. Les dérivés de ces peptides peuvent par exemple porter des modifications destinées à faciliter leur synthèse et/ou à améliorer leur stabilité.

A titre d'exemple de tels dérivés, on citera les peptides incluant des acides aminés dont les groupements carboxyl sont estérifiés ou transformés en groupements amide. Dans le cas du peptide $\beta60\text{-}74_{Cit\text{-}NH2}$, un dérivé de ce dernier peut être le peptide dit $\beta60\text{-}74_{Cit}$ de séquences d'acides aminés SEQ ID NO : 14 dans lequel le résidu citrullyl C-terminal n'est pas amidé.

Les dérivés des peptides peuvent également comprendre des peptides incluant des acides aminés dont un groupement aminé est par exemple méthylé, homocitrulliné, carbamylé ou acétylé.

Un exemple est le peptide $_{Ac}\text{-}\alpha36\text{-}50_{Cit}$ qui est un dérivé acétylé en N-terminal du peptide $\alpha36\text{-}50_{Cit}$. Les peptides $\alpha36\text{-}50_{Cit}$ ou $_{Ac}\text{-}\alpha36\text{-}50_{Cit}$ peuvent être utilisés comme une variante l'un de l'autre dans la présente invention. Toutefois, le peptide $_{Ac}\text{-}\alpha36\text{-}50_{Cit}$ est préféré.

La composition peut comprendre au moins 1, 2, 3, 4 ou 5 peptides choisis dans le groupe consistant en $\alpha171\text{-}185_{Cit}$, $\alpha621\text{-}635_{Cit}$, $\beta60\text{-}74_{Cit\text{-}NH2}$ et $_{Ac}\text{-}\alpha36\text{-}50Cit$ et/ou $\alpha36\text{-}50_{Cit}$, ou des dérivés ou fragments de ceux-ci.

De préférence, la composition selon l'invention peut comprendre au moins 1, 2, 3 ou 4 peptides choisis dans le groupe consistant en $_{Ac}\text{-}\alpha36\text{-}50Cit$, $\alpha171\text{-}185_{Cit}$, $\alpha621\text{-}635_{Cit}$ et $\beta60\text{-}74_{Cit\text{-}NH2}$ ou des dérivés ou fragments de ceux-ci.

La composition peut comprendre 1, 2, 3, 4 ou 5 peptides choisis dans le groupe consistant en $\alpha171\text{-}185_{Cit}$, $\alpha621\text{-}635_{Cit}$ $\beta60\text{-}74_{Cit\text{-}NH2}$ et $\alpha36\text{-}50_{Cit}$ et/ou $_{Ac}\text{-}\alpha36\text{-}50_{Cit}$, ou des dérivés ou fragments de ceux-ci.

De préférence, la composition comprend 1, 2, 3, 4 ou 5 peptides choisis dans le groupe consistant en $_{Ac}\text{-}\alpha36\text{-}50_{Cit}$, $\alpha171\text{-}185_{Cit}$, $\alpha621\text{-}635_{Cit}$ et $\beta60\text{-}74_{Cit\text{-}NH2}$ ou des dérivés ou fragments de ceux-ci.

Plus préférablement, la composition comprend les 4 peptides : $_{Ac}\text{-}\alpha36\text{-}50_{Cit}$, $\alpha171\text{-}185_{Cit}$, $\alpha621\text{-}635_{Cit}$ et $\beta60\text{-}74_{Cit\text{-}NH2}$.

Encore plus préférablement, la composition consiste en $_{Ac}\text{-}\alpha36\text{-}50_{Cit}$, $\alpha171\text{-}185_{Cit}$, $\alpha621\text{-}635_{Cit}$ et $\beta60\text{-}74_{Cit\text{-}NH2}$.

La composition peut également consister en 1, 2, 3 ou 4 peptides choisis dans le groupe consistant en $_{Ac}\text{-}\alpha36\text{-}50_{Cit}$, $\alpha171\text{-}185_{Cit}$, $\alpha621\text{-}635_{Cit}$ et $\beta60\text{-}74_{Cit\text{-}NH2}$ ou des dérivés ou fragments de ceux-ci.

La composition peut comprendre en outre le peptide, dit $\alpha501\text{-}515_{Cit}$, ayant la séquence d'acides aminés $SGIGTLDGFX_1HX_2HPD$ (SEQ ID NO : 10) où $X_1$ et $X_2$ représentent chacun un résidu citrullyl ou des dérivés ou fragments de celui-ci. Aussi, selon un mode de réalisation, la composition comprend et/ou consiste en au moins 1, 2, 3, 4, 5 ou 6 peptides ou 1, 2, 3, 4, 5 ou 6 peptides choisis dans le groupe consistant en $_{Ac}\text{-}\alpha36\text{-}50_{Cit}$ et/ou $\alpha36\text{-}50Cit$, $\alpha171\text{-}185_{Cit}$, $\alpha621\text{-}635_{Ci}$ , $\beta60\text{-}74_{Cit\text{-}NH2}$ et $\alpha501\text{-}515_{Cit}$ ou des dérivés ou fragments de ceux-ci.

Le profil de réactivité des AAPC diffère en fonction des patients. Ainsi les inventeurs ont montré que la plupart des plasmas testés étaient réactifs au(x) peptide(s) $\beta60\text{-}74_{Cit\text{-}NH2}$ et/ou $_{Ac}\text{-}\alpha36\text{-}50_{Cit}$.

Aussi, selon un mode de réalisation, la composition comprend au moins les peptides $\beta60\text{-}74_{Cit\text{-}NH2}$ et/ou $_{Ac}\text{-}\alpha36\text{-}50_{Cit}$ (ou en variante $\alpha36\text{-}50_{Cit}$).

Il existe toutefois des plasmas qui ne sont pas réactifs à ces peptides mais le sont à d'autres peptides. Il est donc préférable de combiner le maximum de peptides pour couvrir le maximum de types de profils de réactivité des plasmas.

De préférence, la composition comprend donc au moins les peptides $\alpha171\text{-}185_{Cit}$, $\alpha621\text{-}635_{Cit}$, $\beta60\text{-}74_{Cit\text{-}NH2}$ et $_{Ac}\text{-}\alpha36\text{-}50_{Cit}$ (ou en variante $\alpha36\text{-}50_{Cit}$). Plus préférablement elle comprend les peptides $\alpha171\text{-}185_{Cit}$, $\alpha621\text{-}635_{Cit}$ $\beta60\text{-}74_{Cit\text{-}NH2}$ et $_{Ac}\text{-}\alpha36\text{-}50_{Cit}$ (ou en variante $\alpha36\text{-}50_{Cit}$) et optionnellement le peptide $\alpha501\text{-}515_{Cit}$.

La présente invention concerne l'utilisation de la composition telle que définie ci-dessus dans la fabrication d'une colonne d'aphérèse, préférablement une colonne d'aphérèse destinée au traitement ou à la prévention de toute maladie auto-immune à auto-anticorps anti-protéines citrullinées. La maladie auto-immune à auto-anticorps anti-protéines citrullinées peut être choisie dans le groupe constitué du syndrome de Sjögren, de l'arthrite juvénile idiopathique et de la polyarthrite rhumatoïde. La maladie auto-immune à auto-anticorps anti-protéines citrullinées est de préférence la polyarthrite rhumatoïde.

Colonne d'aphérèse

La présente invention concerne également une colonne d'aphérèse chargée d'un support solide comprenant la composition telle que définie ci-dessus dont le ou les peptides sont immobilisés directement ou indirectement sur le support.

Le terme "chargé" signifie que la colonne porte ou contient le support solide de manière à ce qu'un liquide tel que du sang ou un produit sanguin puisse circuler à travers la colonne en contact avec le support solide.

De manière générale, l'aphérèse est une technique dans laquelle on fait circuler *ex-vivo* (extracorporellement) du sang ou un produit sanguin provenant d'un sujet au travers d'un dispositif médical qui modifie ce dernier par l'ajout le retrait et/ou le remplacement de composant(s) avant de le réinjecter au sujet. On parle plus précisément de plasmaphérèse lorsque le produit traité par aphérèse est du plasma. Des plasmaphérèses ont notamment été décrites pour désensibiliser des candidats à une transplantation rénale ABO incompatible ; ces candidats possédaient des anticorps anti-ABO qui compromettaient la tolérance du greffon ABO incompatible (L. Rostaing et al., Treatment of large plasma volumes using specific immunoadsorption to desensitize ABO-incompatible kidney transplant candidates, J. Nephropathology. 2016 ;5(3) :90-97).

Ainsi la colonne d'aphérèse selon l'invention permet de retirer les AAPC du sang ou d'un produit sanguin tel que le plasma d'un sujet.

Dans une méthode d'aphérèse classique, le sang est prélevé directement au niveau d'une veine ou d'une artère d'un sujet. Dans certains modes de réalisation, le sang est séparé en un ou plusieurs produits sanguins (par exemple une fraction solide comprenant les globules rouges et blancs, les plaquettes et une fraction liquide telle que le plasma). Un composant (par exemple des auto-anticorps) est retiré d'un des produits sanguins (par exemple le plasma). Eventuellement, les produits sanguins sont combinés. Le sang peut alors être réinjecté dans la veine ou l'artère du sujet.

La présente invention concerne notamment une colonne d'aphérèse chargée d'un support solide comprenant au moins 1, au moins 2, au moins 3, au moins 4 ou au moins 5 ou 1, 2, 3, 4 ou 5 peptide(s) choisi(s) dans le groupe consistant en :

- le peptide, dit $\alpha$171-185$_{Cit}$, ayant la séquence d'acides aminés VDIDIKIX$_1$SCX$_2$GSCS (SEQ ID NO : 8) où X$_1$ et X$_2$ représentent chacun un résidu citrullyl,
- le peptide, dit $\alpha$621-635$_{Cit}$, ayant la séquence d'acides aminés X$_1$GHAKSX$_2$PVX$_3$GIHTS (SEQ ID NO : 12) où X$_1$, X$_2$ et X$_3$ représentent chacun un résidu citrullyl
- le peptide, dit $\beta$60-74$_{Cit-NH2}$, ayant la séquence d'acides aminés X$_1$PAPPPISGGGYX$_2$AX$_3$ (SEQ ID NO: 15) où X$_1$ et X$_2$ représentent chacun un résidu citrullyl et X$_3$ représente un dérivé citrullyl avec un groupement carboxamide à la place du groupement carboxyle et
- le peptide, dit $_{Ac}$-$\alpha$36-50$_{Cit}$, ayant la séquence d'acides aminés GPX$_1$VVEX$_2$HQSACKDS (SEQ ID NO : 6) où le résidu G en N-terminal est acétylé et où X$_1$ et X$_2$ représentent chacun un résidu citrullyl et/ou le peptide, dit $\alpha$36-50$_{Cit}$, ayant la séquence d'acides aminés GPX$_1$VVEX$_2$HQSACKDS (SEQ ID NO : 5) où X$_1$ et X$_2$ représentent chacun un résidu citrullyl,

ou des dérivés ou fragments de ceux-ci,
dont le ou les peptides sont immobilisés directement ou indirectement sur le support.

Le support solide de la colonne d'aphérèse peut également comprendre le peptide, dit $\alpha$501-515$_{Cit}$, ayant la séquence d'acides aminés SGIGTLDGFX$_1$HX$_2$HPD (SEQ ID NO : 10) où X$_1$ et X$_2$ représentent chacun un résidu citrullyl ou des dérivés ou fragments de celui-ci.

Plus préférablement, le support solide de la colonne d'aphérèse comprend les 4 peptides : $_{Ac}$-$\alpha$36-50$_{Cit}$, $\alpha$171-185$_{Cit}$, $\alpha$621-635$_{Cit}$ et $\beta$60-74$_{Cit-NH2}$.

La présente invention concerne également un ensemble de colonnes comprenant au moins 1, au moins 2, au moins 3, au moins 4 ou au moins 5 ou 1, 2, 3, 4 ou 5 colonnes d'aphérèse, chaque colonne d'aphérèse chargée d'un support solide comprenant un peptide choisi dans le groupe consistant en :

- le peptide, dit $\alpha$171-185$_{Cit}$, ayant la séquence d'acides aminés VDIDIKIX$_1$SCX$_2$GSCS (SEQ ID NO : 8) où X$_1$ et X$_2$ représentent chacun un résidu citrullyl,
- le peptide, dit $\alpha$621-635$_{Cit}$, ayant la séquence d'acides aminés X$_1$GHAKSX$_2$PVX$_3$GIHTS (SEQ ID NO : 12) où X$_1$, X$_2$ et X$_3$ représentent chacun un résidu citrullyl
- le peptide, dit $\beta$60-74$_{Cit-NH2}$, ayant la séquence d'acides aminés X$_1$PAPPPISGGGYX$_2$AX$_3$ (SEQ ID NO: 15) où X$_1$ et X$_2$ représentent chacun un résidu citrullyl et X$_3$ représente un dérivé citrullyl avec un groupement carboxamide à la place du groupement carboxyle et
- le peptide, dit $_{Ac}$-$\alpha$36-50Cit, ayant la séquence d'acides aminés GPX$_1$VVEX$_2$HQSACKDS (SEQ ID NO : 6) où le résidu G en N-terminal est acétylé et où X$_1$ et X$_2$ représentent chacun un résidu citrullyl et/ou -le peptide, dit $\alpha$36-50$_{Cit}$, ayant la séquence d'acides aminés GPX$_1$VVEX$_2$HQSACKDS (SEQ ID NO: 5) où X$_1$ et X$_2$ représentent chacun un résidu citrullyl

ou des dérivés ou fragments de ceux-ci,
dont le ou les peptides sont immobilisés directement ou indirectement sur le support.

Selon un mode de réalisation, l'ensemble de colonnes d'aphérèse comprend 4 colonnes d'aphérèse, chacune étant chargée d'un support solide comprenant respectivement le peptide $_{Ac}$-$\alpha$36-50$_{Cit}$, le peptide $\alpha$171-185$_{Cit}$, le peptide $\alpha$621-635$_{Cit}$ et le peptide $\beta$60-74$_{Cit-NH2}$. Selon un mode de réalisation, l'ensemble de colonnes d'aphérèse peut comprendre en outre le peptide $\alpha$501-515$_{Cit}$.

Les peptides selon l'invention sont préférablement des peptides de synthèse.

Les peptides selon l'invention peuvent être biotinylés par des procédés connus dans la technique. Dans des modes de réalisation spécifiques, le ou les peptides sont biotinylés via un groupe espaceur. L'espaceur permet d'éloigner le peptide citrulliné du support solide et en faciliter la reconnaissance par les AAPC. Tout espaceur adapté qui permet le maintien des propriétés de liaison du peptide selon l'invention aux AAPC peut être utilisé dans l'invention. Dans des modes de réalisation spécifiques, l'espaceur est un polyéthylène glycol (PEG) ou un acide aminohexanoïque (AHX).

Le support solide pour immobiliser les peptides de l'invention est connu dans la technique. Le support solide est préférentiellement dans un matériau qui n'active pas les cellules sanguines. Il est préférable d'utiliser un support traité avec un agent anticoagulant, par exemple un support hépariné. Alternativement, le sang du patient peut être traité avec un anticoagulant tel que l'héparine avant l'application sur le support.

Le support solide peut être en polymères tels que des polysaccharides, de préférence de haut poids moléculaire par exemple de 100kDa ou plus, tel que l'agarose ou la cellulose. Le polysaccharide du support peut être réticulé ou non réticulé. D'autres polymères tels que le polystyrène carboxylé peuvent être utilisés. Les supports solides peuvent être sous la forme de billes magnétiques ou de verre.

Le support solide peut être poreux ou non poreux. Il peut être sous la forme de particules qui peuvent être sphériques ou irrégulières. La taille moyenne particules peut aller par exemple de 10 $\mu$m à 2 mm, de préférence de 30$\mu$m à 100$\mu$m.

Le support peut être traité avec un agent anticoagulant comme l'héparine.

Des procédés d'immobilisation de peptides sur un support solide sont connus. Ainsi un peptide selon l'invention peut être immobilisé sur le support de manière directe ou indirecte. Avantageusement, le ou les peptides choisis dans le groupe consistant en $\alpha171-185_{Cit}$, $\alpha621-635_{Cit}$ et $\beta60-74_{Cit-NH2}$, et optionnellement le peptide $\alpha501-515_{Cit}$, sont immobilisés directement ou indirectement sur le support solide par leur extrémité N-terminale et/ou -le(s) peptide(s) $_{Ac}$-$\alpha36-50$Cit et/ou $\alpha36-50_{Cit}$ sont immobilisés directement ou indirectement au support solide par leur extrémité C-terminale.

De préférence, les peptides en $\alpha171-185_{Cit}$, $\alpha621-635_{Cit}$ et $\beta60-74_{Cit-NH2}$, et optionnellement le peptide $\alpha501-515_{Cit}$, sont immobilisés directement ou indirectement sur le support solide par leur extrémité N-terminale et le peptide $_{Ac}$-$\alpha36-50_{Cit}$ (ou en variante $\alpha36-50_{Cit}$) est immobilisé directement ou indirectement sur le support solide par son extrémité C-terminale.

L'immobilisation indirecte peut être réalisée au moyen d'un composé, dit composé intermédiaire, approprié. Un procédé préféré d'immobilisation indirecte d'un peptide repose sur l'interaction entre la biotine et un composé intermédiaire tel que l'avidine ou la streptavidine. Ainsi, la biotinylation du peptide et l'utilisation de l'avidine ou la streptavidine immobilisée sur le support solide permettent une fixation fiable des peptides au support solide. Spécifiquement, le procédé peut comprendre la fourniture du peptide selon l'invention sous forme biotinylée, la fourniture d'un support solide ayant de la streptavidine (ou en variante de l'avidine) immobilisée à sa surface, la mise en contact du support avec une solution aqueuse de peptide(s) selon l'invention biotinylé(s) et le rinçage du support avec un solvant aqueux.

Aussi, selon un mode de réalisation préféré, le support solide comprend alors de la streptavidine ou de l'avidine immobilisée directement sur celui-ci. Le ou les peptides $_{Ac}$-$\alpha36-50_{Cit}$ (ou en variante $\alpha36-50_{Cit}$), $\alpha171-185_{Cit}$, $\alpha621-635_{Cit}$ et $\beta60-74_{Cit-NH2}$, et optionnellement le peptide $\alpha501-515_{Cit}$, sont biotinylés et liés à la streptavidine (ou l'avidine). Les peptides citrullinés peuvent être biotinylés en C-terminal ou en N-terminal. De préférence, les peptides $\alpha171-185_{Cit}$, $\alpha621-635_{Cit}$ et $\beta60-74_{Cit-NH2}$, et optionnellement le peptide $\alpha501-515_{Cit}$, sont biotinylés en N-terminal et le peptide $_{Ac}$-$\alpha36-50_{Cit}$ (ou en variante $\alpha36-50_{Cit}$) est biotinylé en C-terminal. Les peptides $_{Ac}$-$\alpha36-50_{Cit}$ (ou en variante $\alpha36-50_{Cit}$), $\alpha171-185_{Cit}$, $\alpha621-635_{Cit}$ et $\beta60-74_{Cit-NH2}$, et optionnellement le peptide $\alpha501-515_{Cit}$, peuvent être biotinylés via un groupe espaceur, par exemple de l'acide aminohexanoïque (AHX) ou en variante du PEG. Outre l'affinité entre la biotine et l'avidine ou la streptavidine, les interactions anticorpsantigène peuvent également être utilisées pour l'immobilisation indirecte de peptides sur un support.

En variante, les peptides peuvent être immobilisés directement sur un support solide en utilisant des techniques de bio-conjugaison telles que par exemple, l'immobilisation directe de peptides sur des supports solides activés au bromure de cyanogène via des fonctionnalités amino dans la séquence primaire du peptide ou l'immobilisation directe de peptides sur des supports solides contenant des époxydes activés via des fonctionnalités carboxyl dans la séquence primaire du peptide. En variante, la chimie «Click» peut être utilisée pour immobiliser des peptides sur des supports solides, le peptide et le support étant modifiés avec les fonctionnalités chimiques mutuellement réactives appropriées (azides et alcynes). Dans d'autres modes de réalisation, la chimie de ligature de Staudinger peut être utilisée pour immobiliser des peptides modifiés de manière appropriée sur les supports solides dérivés de manière appropriée.

Méthode de traitement

La présente invention concerne également une composition telle que définie précédemment pour son utilisation dans une colonne d'aphérèse, préférablement une colonne d'aphérèse destinée au traitement ou à la prévention d'une maladie auto-immune à auto-anticorps anti-protéines citrullinées.

Par maladie auto-immune à auto-anticorps anti-protéines citrullinées, on entend une maladie dans laquelle l'organisme d'un sujet développe des anticorps dirigés contre des protéines citrullinées de ce même sujet. La maladie auto-immune à auto-anticorps anti-protéines citrullinées peut être choisie notamment dans le groupe constitué du syndrome de Sjögren, de l'arthrite juvénile idiopathique et de la polyarthrite rhumatoïde. De préférence, la maladie auto-immune à auto-anticorps anti-protéines citrullinées est la polyarthrite rhumatoïde.

La présente invention concerne également une composition telle que définie précédemment pour son utilisation dans le traitement ou la prévention, préférablement par aphérèse, d'une maladie auto-immune à auto-anticorps anti-protéines citrullinées, de préférence la polyarthrite rhumatoïde, où le ou les peptides de la composition sont immobilisés sur un support solide.

Préférablement, le support solide est chargé sur une colonne d'aphérèse.

De préférence, le traitement ou la prévention d'une maladie auto-immune à auto-anticorps anti-protéines citrullinées comprend le traitement *in vitro* du sang d'un patient atteint de ladite maladie auto-immune avec une colonne d'aphérèse chargée d'un support solide comprenant la composition telle que définie précédemment.

La présente invention concerne également une composition telle que définie précédemment pour son utilisation dans une colonne d'aphérèse, préférablement une colonne d'aphérèse destinée au traitement de la polyarthrite rhumatoïde.

La présente invention concerne également l'utilisation d'une colonne d'aphérèse telle que définie précédemment, en particulier l'utilisation de cette colonne d'aphérèse dans le traitement ou la prévention d'une maladie auto-immune à auto-anticorps anti-protéines citrullinées et plus préférablement de la polyarthrite rhumatoïde.

La présente demande décrit également une méthode de traitement ou de prévention par aphérèse, chez un sujet le nécessitant dans laquelle est une colonne d'aphérèse telle que définie précédemment est utilisée.

La présente demande décrit aussi une méthode pour épurer des anticorps anti-peptides citrullinés (AAPC) du sang ou d'un produit sanguin d'un sujet, comprenant :

- la fourniture d'une colonne d'aphérèse telle que définie précédemment
- la mise en contact de cette colonne avec le sang ou un produit sanguin du sujet de manière à dépléter des AAPC le sang ou le produit sanguin du sujet.

Le sujet traité par l'invention est de préférence un mammifère, plus préférablement un être humain.

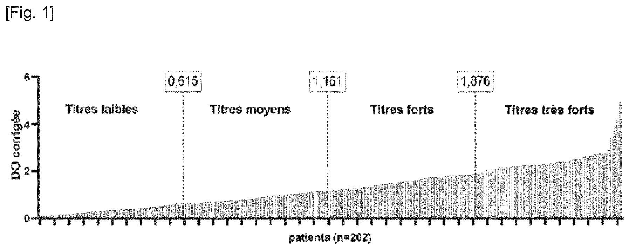

Selon un mode de réalisation préféré, le sujet traité selon l'invention est un sujet ayant une forme sévère de polyarthrite rhumatoïde. Le sujet peut également être un sujet à haut titre d'anticorps anti-protéines/peptides citrullinés (AAPC). Par sujet à haut titre d'AAPC, on entend des sujets présentant une DO ≥0.9 lors d'un dosage des AAPC par un test AhFibA. Une DO≥0.9 en test AhFibA correspond aux 40% de patients atteints de polyarthrite rhumatoïde ayant les taux d'AAPC les plus élevés (figure 1).

L'invention sera en outre illustrée par les figures et exemples suivants.

## Brève description des figures

**[Fig. 1]** Répartition des titres AhFibA déterminés sur des échantillons de sérum provenant de 202 patients atteints de polyarthrite rhumatoïde AhFibA-positifs. Les valeurs-seuils de Densité Optique (la DO corrigée définit le Titre) séparant les quartiles sont notées.

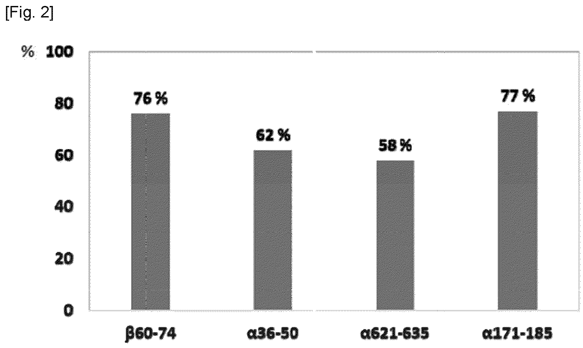

**[Fig. 2]** Pourcentage de sérums réactifs en ELISA vis-à-vis des 4 peptides $\beta60\text{-}74_{Cit\text{-}NH2}$, $_{Ac}\text{-}\alpha36\text{-}50_{Cit}$, $\alpha621\text{-}635_{Cit}$ et $\alpha171\text{-}185_{Cit}$, parmi 202 sérums provenant de patients atteints de polyarthrite rhumatoïde et AhFibA-positifs.

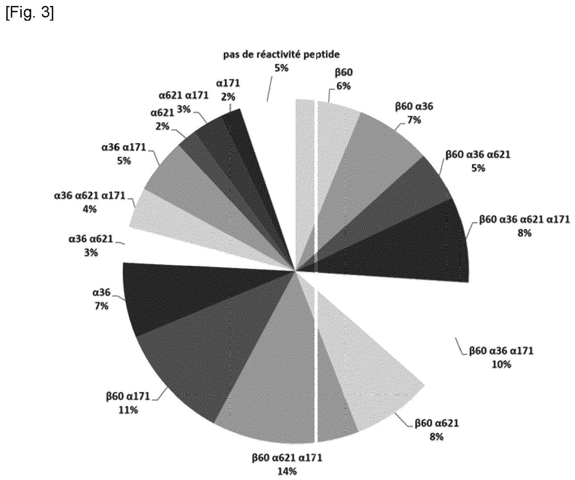

**[Fig. 3]** Répartition en fonction de leur profil de réactivité vis-à-vis des 4 peptides $\beta60\text{-}74_{Cit\text{-}NH2}$, $_{Ac}\text{-}\alpha36\text{-}50_{Cit}$, $\alpha621\text{-}635_{Cit}$ et $\alpha171\text{-}185_{Cit}$, de 202 sérums AhFibA-positifs provenant de patients atteints de polyarthrite rhumatoïde.

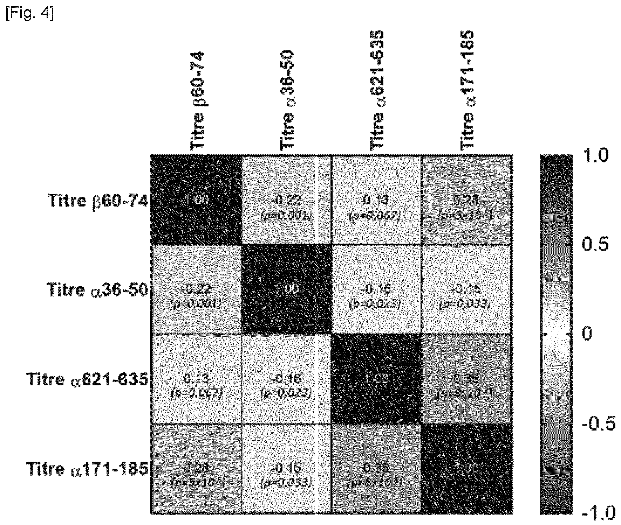

**[Fig. 4]** Corrélations entre les titres des anticorps dirigés contre les 4 peptides $\beta60\text{-}74_{Cit\text{-}NH2}$, $_{Ac}\text{-}\alpha36\text{-}50_{Cit}$, $\alpha621\text{-}635_{Cit}$ et $\alpha171\text{-}185_{Cit}$, pour les 202 sérums testés. A chaque intersection sont notés coefficient de Spearman et (p) de la corrélation.

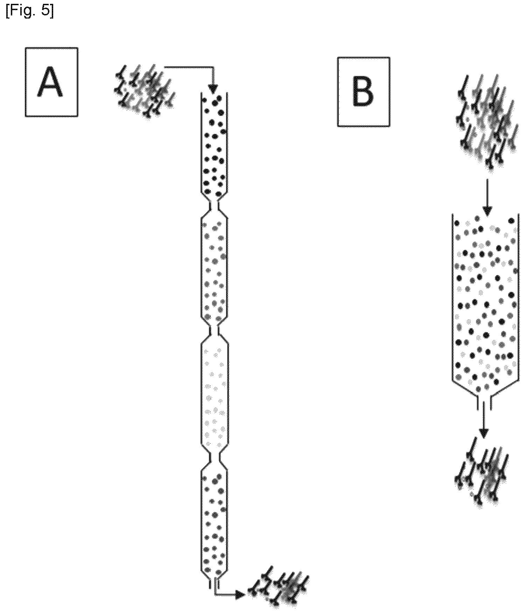

**[Fig. 5]** Deux types de chromatographies ont été utilisés pour purifier simultanément les anticorps anti-$\beta60\text{-}74_{Cit\text{-}NH2}$, ant -$_{Ac}\text{-}\alpha36\text{-}50_{Cit}$, anti-$\alpha621\text{-}635_{Cit}$ et anti-$\alpha171\text{-}185_{Cit}$ : soit 4 colonnes mono-peptidiques reliées en série (A), soit une seule colonne contenant un mélange équilibré des 4 matrices mono-peptidiques (B).

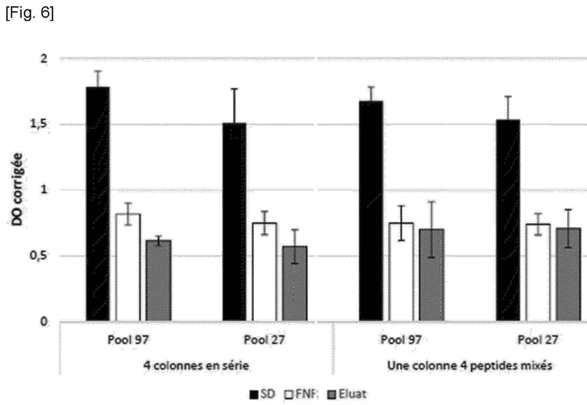

**[Fig. 6]** Chromatographies réalisées à partir de pools de sérums de patients PR, soit sur les 4 colonnes montées en série, chargées respectivement avec les peptides $\beta60\text{-}74_{Cit\text{-}NH2}$, $_{Ac}\text{-}\alpha36\text{-}50_{Cit}$, $\alpha621\text{-}635_{Cit}$ et $\alpha171\text{-}185_{Cit}$ (histogrammes de gauche), soit sur une colonne contenant le mélange des 4 peptides (histogrammes de droite). Le Pool 97 était constitué de 97 échantillons de sérum de même volume provenant de patients présentant des titres croissants en AAPC représentatifs de ceux rencontrés dans une population de patients PR ; le Pool 27 était constitué de 27 échantillons de sérum de même volume provenant de patients présentant des hauts titres d'AAPC (DO ≥ 1,5). Les pools (sérum de départ : SD - barre noire), les fractions non retenues (FNR - barre blanche) et les éluats (barre grise), ont été analysés en ELISA AhFibA (n≥4), les résultats sont exprimés en DO corrigées.

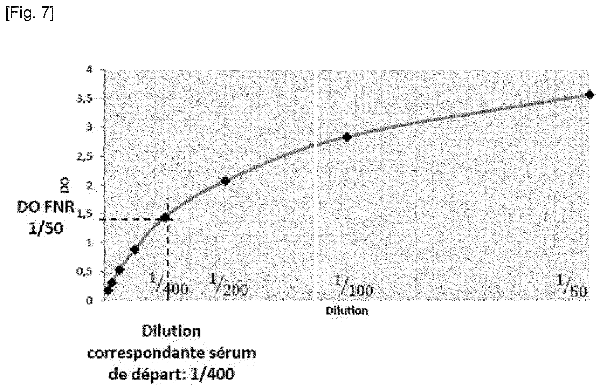

**[Fig. 7]** Méthode de calcul du taux d'épuration d'un sérum après chromatographie. La fraction non retenue est analysée en ELISA AhFibA et la DO obtenue comparée à celles résultant de la dilution progressive du sérum de départ (courbe de référence). Le rapport des dilutions permet de déterminer le taux d'épuration. Dans cet exemple, 1/50 vs 1/400 : 8 fois moins d'anticorps dans la FNR, c'est-à-dire 12,5% des anticorps présents dans le sérum de départ. 87,5 % des AAPC ont donc été épurés.

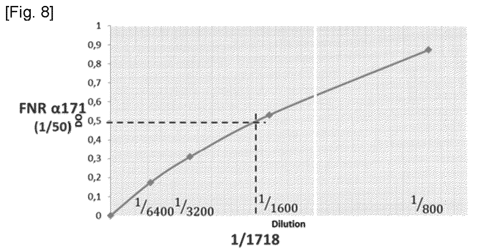

**[Fig. 8]** Calcul du taux d'épuration en AAPC d'un sérum (n° 41) après chromatographie sur les 4 colonnes chargées respectivement avec les peptides $\beta60\text{-}74_{Cit\text{-}NH2}$, $_{Ac}\text{-}\alpha36\text{-}50_{Cit}$, $\alpha621\text{-}635_{Cit}$, $\alpha171\text{-}185_{Cit}$ montées en série. La fraction non retenue a été analysée en ELISA AhFibA et la DO obtenue comparée à celles résultant de la dilution du sérum de départ (courbe de référence). Le rapport des dilutions 1/50 vs 1/1718 permet de déterminer le taux d'épuration : 98% des AAPC ont été épurés.

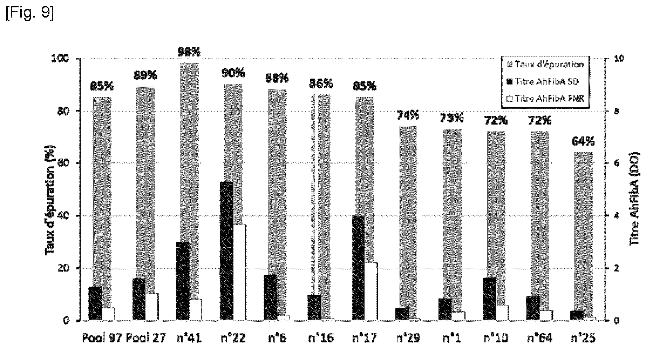

**[Fig. 9]** Chromatographies réalisées à partir du Pool 97, du Pool 27 et de 10 sérums individuels, provenant de patients PR, sur les 4 colonnes chargées respectivement avec les peptides $\beta60\text{-}74_{Cit\text{-}NH2}$, $_{Ac}\text{-}\alpha36\text{-}50_{Cit}$, $\alpha621\text{-}635_{Cit}$ et $\alpha171\text{-}185_{Cit}$ et montées en série. Pour chaque pool et sérum individuel, les sérums de départ (SD - barres noires) et les fractions non retenues (FNR - barres blanches) ont été analysés en ELISA AhFibA (n≥4) : leurs titres sont exprimés en DO corrigées. Les taux d'épuration (%) ont été calculés pour tous les échantillons chromatographiés (barres grises).

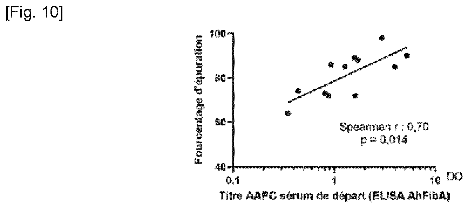

**[Fig. 10]** Corrélation entre le titre en AAPC (ELISA AhFibA) du sérum de départ (avant chromatographie) et le taux d'épuration obtenu après chromatographie sur les 4 colonnes en série, chargées respectivement avec les peptides $\beta60\text{-}74_{Cit\text{-}NH2}$, $_{Ac}\text{-}\alpha36\text{-}50_{Cit}$, $\alpha621\text{-}635_{Cit}$ et $\alpha171\text{-}185_{Cit}$. La corrélation est très significative (p=0,014) : l'épuration est d'autant plus efficace que les sérums présentent un titre d'AAPC élevé.

## Exemples

### Matériel et méthodes

### Echantillons de sérum

Des échantillons de sérum ont été obtenus à partir de 202 patients atteints de polyarthrite rhumatoïde présentant une positivité au test ELISA « AhFibA » qui permet de détecter et titrer les AAPC en utilisant le fibrinogène humain citrulliné comme immunosorbant.

### Peptides de synthèse

Des peptides correspondant aux résidus 36 à 50, 171 à 185 et 621 à 635 de la chaîne $\alpha$ du fibrinogène humain (NP_068657 - Isoforme 2) et des résidus 60 à 74 de sa chaîne $\beta$ (AAA18024), ont été synthétisés.

Les peptides ont été synthétisés dans leur forme citrullinée (par substitution systématique de tous les résidus arginyl par un résidu citrullyl) ou native non citrullinée (résidus arginyl). Les peptides $\beta60\text{-}74$ et $\beta60\text{-}74_{Cit}$ ont été synthétisés avec un carboxamide à la place du groupe carboxyle terminal afin d'obtenir les peptides $\beta60\text{-}74_{NH2}$ et $\beta60\text{-}74_{Cit\text{-}NH2}$.

Les peptides $\alpha36\text{-}50$, et $\alpha36\text{-}50_{Cit}$ ont été synthétisés avec un acétyl côté N-terminal afin d'obtenir les peptides $_{Ac}\text{-}\alpha36\text{-}50$ et $_{Ac}\text{-}\alpha36\text{-}50_{Cit}$.

Les peptides ont été biotinylés en C-terminal d'un espaceur aminohexanoyl pour le peptide $_{Ac}\text{-}\alpha36\text{-}50Cit$ et en N-terminal du même espaceur, pour les peptides $\alpha171\text{-}185_{Cit}$, $\alpha621\text{-}635_{Cit}$ et $\beta60\text{-}74_{Cit\text{-}NH2}$.

### Tests ELISA

Les antigènes ou immunosorbants (fibrinogène citrulliné ou peptides citrullinés et non citrullinés), solubilisés en PBS (1.5mM $KH_2PO4$ SIGMA 795488 ; 7mM $K_2HPO_4$ SIGMA P3786 ; 0,15M NaCl SIGMA 31434 ; pH 7.2) aux concentrations de 5 et 10$\mu$g/mL, sont adsorbés dans les puits de plaques de microtitration (MAXISORP NUNC 2023-09) par incubation une nuit à 4°C, ou bien utilisés en système avidine-biotine : avidine 5$\mu$g/mL en PBS incubée une nuit à 4°C puis, après lavage, peptide biotinylé à 10$\mu$g/mL en PBS, incubé 1h à 4°C. Après blocage en PBS 2% BSA (Sigma A3059) pendant 1h à 4°C et lavage en PBS 0,1% Tween®20 (SIGMA P1379), les échantillons à tester sont déposés dilués au 1/50 (1/100 en système Avidine-Biotine) ou à des dilutions corrigées équivalentes, en PBS 2% BSA 2MNaCl. Après lavage, l'anticorps secondaire (IgG de chèvre anti-fragment Fc d'IgG humaine couplé à la peroxydase de raifort (Horseradish Peroxidase ; Southern Biotech 2040-05) est incubé 1h à 4°C. Après lavage, la révélation est effectuée avec une solution d'Ortho-Phénylène Diamine dihydrochloride (Sigma P2788), 0,03% $H_2O_2$ (Sigma H1009), en tampon citrate/H₃PO₄ pH5 (0,05 M acide citrique Sigma C0759 ; 0,1 M Na2HPO4 Prolabo 28026292) pendant 5 minutes à température ambiante, puis stoppée avec une solution d'H₂SO₄ 6N (Sigma 07208). La densité optique (DO) est lue à 492 nm avec un lecteur de plaques Multiskan Fc (ThermoScientific).

Conditions de réalisation des tests ELISA

**[Tableau 2]**

|  | Concentration de l'immunosorbant | Dilution échantillon | Dilution anticorps secondaire |
|---|---|---|---|
| AhFibA | 5 μg/mL | 1/50 | 1/15000 |
| Anti-β60-74 | 10 μg/mL | 1/50 | 1/2500 |
| Anti-α36-50 | 10 μg/mL | 1/50 | 1/2500 |
| Anti-α621-635 | 10 μg/mL | 1/50 | 1/2500 |
| Anti-α171-185biot | 10 μg/mL | 1/100 | 1/2500 |

Expression des résultats des tests ELISA : pour les peptides, est prise en compte la variation de DO entre la réactivité du peptide citrulliné et la réactivité du peptide non citrulliné, chaque réactivité étant préalablement retranchée de son blanc (DO en absence d'échantillon). Ce qui donne la formule suivante :

**[Math 1]**

$$\Delta DO = (DO_{cit} - DO_{blanc\ cit}) - (DO_{noncit} - DO_{blanc\ noncit})$$

A noter que pour le fibrinogène, la forme non citrullinée n'ayant jamais aucune réactivité, il n'est pas nécessaire de l'évaluer. Pour le test AhFibA la valeur de DO est donc simplement retranchée de son blanc (DO en absence d'échantillon).

La ΔDO pour les peptides et la DO pour le fibrinogène citrulliné, sont considérées comme exprimant le titre des échantillons testés.

Les variations inter-essais sont corrigées grâce à une gamme de dilutions d'un pool de sérums de patients testée dans chaque plaque de micro-titration. Ce pool de référence, nommé P97, a été constitué à partir de 97 échantillons de même volume provenant de 97 patients présentant un titre élevé d'AAPC. Les valeurs de DO de cette gamme interne sont comparées à celle de valeurs de référence où chaque point de la gamme est la moyenne de 30 déterminations préalablement effectuées à partir de ce même pool. La méthode des moindres carrés permet ensuite d'obtenir un facteur de correction qui est appliqué à l'ensemble des valeurs de DO obtenues sur la plaque correspondante. Cette DO corrigée correspond au titre de l'échantillon.

**[Math 2]**

$$DO\ corrigée = \frac{\sum X * Y *}{\sum X^2} * DO$$

Avec : X valeurs internes, Y : valeurs de référence

Construction de colonnes de chromatographie

Quatre colonnes HiTrap Streptavidine HP de 1 mL (GE ; 29-0513-24) sont chargées indépendamment avec chacun des 4 peptides β60-74_{Cit-NH2}, Ac-α36-50_{Cit}, α621-635_{Cit} et α171-185_{Cit} biotinylés. Après équilibration de la colonne en PBS, un volume-colonne (1 mL) de solution saturante de peptides biotinylés est injecté à 0,2mL/min, incubé pendant 15 min puis récupéré en sortie de colonne et lyophilisé.

La quantité de peptide restante de la fraction lyophilisée est dosée par chromatographie liquide haute performance (UPLC) ; cette quantité est soustraite à la quantité de départ pour estimer la quantité de peptide liée dans la colonne.

**[Tableau 3]**

| Peptide | Quantité de peptide liée (μmol) |
|---|---|
| β60-74_{Cit-NH2} | 0,83 |

(suite)

| Peptide | Quantité de peptide liée ($\mu$mol) |
|---|---|
| Ac-$\alpha$36-50$_{Cit}$ | 1,1 |
| $\alpha$621-635$_{Cit}$ | 0,87 |
| $\alpha$171-185$_{Cit}$ | 1,7 |

Quatre colonnes ont été chargées avec les quantités respectives de peptides présentées dans le tableau ci-dessus.

Après utilisation pour les tests d'épuration en série, les 4 matrices des colonnes chargées avec les peptides $\beta$60-74$_{Cit-NH2}$, $\alpha$36$_{Ac}$-50 $_{Cit}$, $\alpha$621-635$_{Cit}$ et $\alpha$171-185$_{Cit}$ ont été récupérées et mélangées puis conditionnées dans une colonne vide de 10 mL (GE, C10/10) avec adaptateur de volume (GE, AC10), afin de former une colonne chargée avec les 4 peptides mixés d'un volume final de 4mL.

Epuration

Deux pools représentatifs, P97 (précédemment décrit) et P27, pool de 27 sérums de patients ayant un titre en AAPC supérieur à 1,5, ainsi que des sérums individuels choisis en fonction de leur réactivité sur les 4 peptides $\beta$60-74$_{Cit-NH2}$, $\alpha$36$_{Ac}$ -50 $_{Cit}$, $\alpha$621-635$_{Cit}$ et $\alpha$171-185$_{Cit}$ , ont été dilués au ¼ en PBS avant chromatographie.

Après équilibrage des colonnes en PBS à un débit de 0,5mL/min, un volume de 6mL de pool ou de sérum ainsi dilué a été injecté sur les 4 colonnes montées en série dans l'ordre suivant : colonne chargée en peptide $\beta$60-74$_{Cit-NH2}$ puis $\alpha$36$_{Ac}$ -50 $_{Cit}$, $\alpha$621-635$_{Cit}$ et $\alpha$171-185$_{Cit}$, et ultérieurement sur la colonne contenant les 4 peptides. Les fractions non retenues ont été récupérées. Après lavage en PBS, l'élution des anticorps spécifiques des 4 peptides a été réalisée avec un tampon glycine-HCl (Invitrogen ; 15527-013) 0,2M pH3. Les fractions éluées ont été récupérées et ramenées à pH 7 avec une solution de Tris 2M (Euromedex ; 77-86-1). Sérums de départ, fractions non retenues et éluats, ont ensuite été testés en ELISA après correction du facteur de dilution induit par la chromatographie.

Détermination du pourcentage ou taux d'épuration

Pour chaque pool ou sérum traité, le pourcentage d'épuration a été calculé à partir de l'immunoréactivité résiduelle en AAPC (test ELISA AhFibA) des fractions non retenues, épurées, et d'une gamme de dilutions du pool ou sérum de départ correspondant.

[Math 3]

$$\% \, épuration = \left( 1 - \frac{Dilution \; SD \; correspondant \; à \; DO \; FNR}{Dilution \; expérimentale \; FNR} \right) * 100$$

Un exemple fictif de calcul de pourcentage d'épuration est illustré à la figure 7 où pour obtenir une DO équivalente à celle de la fraction non retenue (testée au 1/50 après correction de la dilution due à la chromatographie) le sérum de départ doit être dilué au 1/400. La concentration en AAPC dans la fraction non retenue est donc 8 fois inférieure à celle du sérum de départ. Un huitième, soit 12,5%, des AAPC du sérum de départ sont présents dans la fraction non retenue et n'ont donc pas été épurés. On en déduit que sept huitièmes des AAPC du sérum de départ, soit 87,5%, ont été épurés : c'est le taux d'épuration.

Profil de réactivité des échantillons de sérum

Les profils de réactivité des 202 échantillons de sérums de patients vis-à-vis du fibrinogène citrulliné et des 4 peptides citrullinés, ont été déterminés par ELISA sur au moins deux essais indépendants, chacun étant réalisé en double. Dans un souci de reproductibilité, seules les plaques de microtitration dont la gamme interne nécessitait l'application d'un facteur de correction compris entre 0,5 et 2 par rapport à la gamme de référence, ont été prises en compte.

**Résultats**

Profils de réactivité en ELISA de sérums de patients atteints de polyarthrite rhumatoïde (PR), vis-à-vis du fibrinogène citrulliné (test AhFibA) et vis-à-vis des 4 peptides immunodominants.

202 sérums de patients PR ont été testés en AhFibA puis classés par ordre de réactivité (DO ou titre) croissante (figure 1). En fonction de leur titre, les patients ont été répartis en quartiles : titres faibles (DO = [0,056 - 0,615]), moyens (DO = [0,615 - 1,161]), forts (DO = [1,161 - 1,876]) et très forts (DO $\geq$ 1,876).

La figure 2 montre les pourcentages de sérums PR qui sont réactifs vis-à-vis de chacun des 4 peptides : 76% sont positifs sur le peptide $\beta 60\text{-}74_{Cit\text{-}NH2}$, confirmant qu'il est bien le plus immunodominant.

La réactivité individuelle des sérums vis-à-vis des 4 peptides est très variable et permet de définir de nombreux profils de réactivité. La fréquence des différents profils observés est montrée sur la figure 3. Parmi ces 202 sérums, 78% sont réactifs contre au moins 2 peptides parmi les 4, et 95% réagissent avec au moins un des 4 peptides. Ces résultats montrent que l'utilisation d'une combinaison des 4 peptides en chromatographie doit permettre de viser un très large spectre de patients.

La recherche de corrélations entre les réactivités des sérums vis-à-vis des différents peptides fait apparaître des corrélations significatives entre les peptides $\beta 60\text{-}74_{Cit\text{-}NH2}$, $\alpha 621\text{-}635_{Cit}$ et $\alpha 171\text{-}185_{Cit}$; celles-ci traduisent un certain degré de réactivité croisée entre ces peptides, néanmoins leur relative faiblesse montre que chaque peptide présente bien une réactivité propre. En revanche, aucune corrélation n'est retrouvée entre $\alpha 36_{Ac}\text{-}50_{Cit}$ et les 3 autres peptides. Le tableau à double entrée présenté en figure 4 visualise les coefficients de Spearman calculés pour toutes les paires de peptides comparées et leur caractère significatif ou non. Ces résultats confortent l'idée que pour développer un système d'épuration visant l'ensemble des AAPC pour le plus grand nombre possible de patients, il est nécessaire d'utiliser conjointement les 4 peptides. C'est donc finalement ce choix qui est fait.

Epuration de pools de sérums de patients PR sur 4 colonnes de chromatographie chargées respectivement avec les peptides $\beta 60\text{-}74_{Cit\text{-}NH2}$, $_{Ac}\text{-}\alpha 36\text{-}50_{Cit}$, $\alpha 621\text{-}635_{Cit}$ et $\alpha 171\text{-}185_{Cit}$ et montées en série, ou bien sur une colonne contenant un mélange des 4 peptides.

Deux systèmes de chromatographie, représentés sur la figure 5, ont été imaginés pour épurer les sérums de patients des AAPC qu'ils contiennent en utilisant les 4 peptides. Le premier système consiste à brancher en série 4 colonnes chargées respectivement avec l'un des 4 peptides $\beta 60\text{-}74_{Cit\text{-}NH2}$, $_{Ac}\text{-}\alpha 36\text{-}50_{Cit}$, $\alpha 621\text{-}635_{Cit}$ et $\alpha 171\text{-}185_{Cit}$ (A), le second consiste à mélanger en quantités équivalentes les 4 matrices mono-peptidiques et à les charger dans une colonne unique (B).

L'efficacité des deux systèmes a été comparée à l'aide de deux pools de sérums de patients PR. Ces conditions ont été choisies parce que plus exigeantes que celles où l'on teste un sérum individuel. En effet, dans un pool contenant de nombreux sérums qui présentent des profils de réactivité différents, le mélange d'AAPC est hautement polyclonal et composé d'AAPC de spécificité antigénique différente.

Un premier pool, P97, est constitué de 97 sérums présentant une répartition des titres AhFibA représentative de celle de la population générale des patients PR. Un second pool, P27, est constitué de 27 sérums de patients présentant des hauts titres en AhFibA (DO >1,5).

La figure 6 montre les résultats de chromatographies réalisées à partir de ces pools, soit sur les 4 colonnes montées en série, chargées respectivement avec les peptides $\beta 60\text{-}74_{Cit\text{-}NH2}$, $_{Ac}\text{-}\alpha 36\text{-}50_{Cit}$, $\alpha 621\text{-}635_{Cit}$ et $\alpha 171\text{-}185_{Cit}$, soit sur une colonne contenant le mélange des 4 peptides. Une proportion importante des AAPC a été absorbée sur les colonnes et peut en être éluée, alors qu'une autre partie, non absorbée, se retrouve dans la fraction non retenue par les colonnes. Il apparaît que les deux systèmes de chromatographie s'avèrent efficaces pour épurer une grande partie des AAPC contenus dans les pools et qu'ils donnent des résultats peu différents.

Par ailleurs, 10 sérums positifs en AAPC ont été choisis en fonction de leurs profils de réactivité vis-à-vis des 4 peptides $\beta 60\text{-}74_{Cit\text{-}NH2}$, $_{Ac}\text{-}\alpha 36\text{-}50_{Cit}$, $\alpha 621\text{-}635_{Cit}$ et $\alpha 171\text{-}185_{Cit}$ (tableau 4 ci-dessous).

**[Tableau 4]**

| N° de sérum | $\beta 60\text{-}74_{Cit\text{-}NH2}$ | $_{Ac}\text{-}\alpha 36\text{-}50_{Cit}$ | $\alpha 621\text{-}635_{Cit}$ | $\alpha 171\text{-}185_{Cit}$ |
|---|---|---|---|---|
| n°1 | +++ | | + | + |
| n°10 | +++ | | | + |
| n°41 | | +++ | | ++ |
| n°16 | | + | +++ | ++ |
| n°29 | | + | +++ | |
| n°25 | ++ | +++ | + | |

(suite)

| N° de sérum | $\beta$60-74$_{Cit-NH2}$ | $_{Ac}$-$\alpha$36-50$_{Cit}$ | $\alpha$621-635$_{Cit}$ | $\alpha$171-185$_{Cit}$ |
|---|---|---|---|---|
| n°64 | +++ | +++ | | |
| n°22 | ++ | | ++ | +++ |
| n°6 | +++ | | +++ | + |
| n°17 | +++ | + | +++ | +++ |
| (+++ : positif fort ; ++ : positif moyen ; + : positif faible ; case vide : négatif) | | | | |

Ces sérums ainsi que les deux pools P97 et P27 ont été passés sur 4 colonnes en série chargées respectivement avec les 4 peptides $\beta$60-74$_{Cit-NH2}$, $_{Ac}$-$\alpha$36-50$_{Cit}$, $\alpha$621-635$_{Cit}$ et $\alpha$171-185$_{Cit}$. Pour l'ensemble des échantillons, le titre en AAPC a diminué fortement après passage sur les colonnes (figure 9).

Un indicateur d'efficacité de l'épuration était nécessaire pour comparer les résultats entre les différentes méthodes chromatographiques et les différents échantillons. La densité optique d'un échantillon analysé en ELISA n'étant pas corrélée de manière linéaire mais exponentielle à la quantité d'AAPC contenue dans cet échantillon, un simple ratio de densités optiques entre le titre du sérum de départ et celui de la fraction non retenue sur les colonnes, ne pouvait pas permettre de calculer un taux d'épuration.

Nous avons donc utilisé une méthode graphique qui consiste à déterminer en ELISA AhFibA les DO d'une série de dilutions du sérum de départ, permettant d'établir une courbe de référence. La DO de la fraction non retenue est déterminée de la même façon, après correction du facteur de dilution lié à la chromatographie. La lecture graphique sur la courbe de référence indique à quelle dilution du sérum de départ correspond la DO de la fraction non retenue. Le pourcentage d'épuration calculé résulte du ratio entre la dilution expérimentale de la fraction non retenue et la dilution correspondante du sérum de départ. Cette méthode graphique de détermination du taux d'épuration est illustrée sur la figure 7. Un exemple de calcul de ce taux pour l'un des sérums traités (sérum n° 41) est montré à la figure 8.

Les taux d'épuration calculés pour les 2 pools et pour les 10 sérums individuels après un seul passage sur les colonnes, sont tous supérieurs à 65% et 7 sur 12, incluant les 2 pools, sont supérieurs à 85%. Pour le sérum n°41, ce taux atteint 98%.

La relation entre le titre en AAPC du sérum de départ et le taux d'épuration obtenu après passage sur les 4 colonnes montées en série, a été étudiée. La figure 10 montre qu'il existe une corrélation forte et significative (r de Spearman : 0,70 ; p = 0,014) entre ces deux paramètres. Plus le titre en AAPC du sérum est élevé, plus efficace est son épuration. Ce résultat, contre-intuitif, résulte du fait que les AAPC de titre élevé présentent une haute affinité et donc se lient plus efficacement à leurs antigènes.

## Revendications

1. Colonne d'aphérèse chargée d'un support solide comprenant une composition qui comprend au moins un peptide choisi dans le groupe consistant en :

    - le peptide, dit $\alpha$171-185$_{Cit}$, ayant la séquence d'acides aminés VDIDIKIX$_1$SCX$_2$GSCS (SEQ ID NO : 8) où X$_1$ et X$_2$ représentent chacun un résidu citrullyl,
    - le peptide, dit $\alpha$621-635$_{Cit}$, ayant la séquence d'acides aminés X$_1$GHAKSX$_2$PVX$_3$GIHTS (SEQ ID NO : 12) où X$_1$, X$_2$ et X$_3$ représentent chacun un résidu citrullyl,
    - le peptide, dit $\beta$60-74$_{Cit-NH2}$, ayant la séquence d'acides aminés X$_1$PAPPPISGGGYX$_2$AX$_3$ (SEQ ID NO: 15) où X$_1$ et X$_2$ représentent chacun un résidu citrullyl et X$_3$ représente un dérivé citrullyl avec un groupement carboxamide à la place du groupement carboxyle et
    - le peptide, dit $_{Ac}$-$\alpha$36-50$_{Cit}$, ayant la séquence d'acides aminés GPX$_1$VVEX$_2$HQSACKDS (SEQ ID NO : 6) où le résidu G en N-terminal est acétylé et où X$_1$ et X$_2$ représentent chacun un résidu citrullyl et/ou le peptide, dit $\alpha$36-50$_{Cit}$, ayant la séquence d'acides aminés GPX$_1$VVEX$_2$HQSACKDS (SEQ ID NO : 5) où X$_1$ et X$_2$ représentent chacun un résidu citrullyl

    dont le ou les peptides sont immobilisés directement ou indirectement sur le support.

2. Colonne d'aphérèse selon la revendication 1 **caractérisée en ce qu'**elle comprend au moins 2 peptides choisis dans le groupe consistant en $\alpha$36-50$_{Cit}$, $_{Ac}$-$\alpha$36-50$_{Cit}$, $\alpha$171-185$_{Cit}$, $\alpha$621-635$_{Cit}$ et $\beta$60-74$_{Cit-NH2}$.

**3.** Colonne d'aphérèse selon la revendication 1 ou 2 **caractérisée en ce qu'**elle comprend les peptides $\alpha171\text{-}185_{Cit}$, $\alpha621\text{-}635_{Cit}$, $\beta60\text{-}74_{Cit\text{-}NH2}$ et le(s) peptide(s) $\alpha36\text{-}50_{Cit}$ et/ou $_{Ac}\text{-}\alpha36\text{-}50_{Cit}$.

**4.** Colonne d'aphérèse selon l'une quelconque des revendications 1 à 3 **caractérisée en ce qu'**elle comprend en outre le peptide $\alpha501\text{-}515_{Cit}$ de séquence d'acides aminés SGIGTLDGFX$_1$HX$_2$HPD (SEQ ID NO : 10) où X$_1$ et X$_2$ représentent chacun un résidu citrullyl.

**5.** Colonne d'aphérèse selon l'une quelconque des revendications 1 à 4 **caractérisée en ce que** :

- le ou les peptides choisis dans le groupe consistant en $\alpha171\text{-}185_{Cit}$, $\alpha621\text{-}635_{Cit}$, et $\beta60\text{-}74_{Cit\text{-}NH2}$, et optionnellement le peptide $\alpha501\text{-}515_{Cit}$, sont immobilisés directement ou indirectement sur le support solide par leur extrémité N-terminale et/ou
- le(s) peptide(s) $_{Ac}\text{-}\alpha36\text{-}50_{Cit}$ et/ou $\alpha36\text{-}50_{Cit}$ sont immobilisés directement ou indirectement sur le support solide par leur extrémité C-terminale.

**6.** Colonne d'aphérèse selon l'une quelconque des revendications 1 à 5 **caractérisée en ce que** le ou les peptides sont immobilisés indirectement sur le support solide au moyen d'un ou plusieurs composés intermédiaires.

**7.** Colonne d'aphérèse chargée d'un support solide comprenant une composition qui comprend au moins deux peptides choisis dans le groupe consistant en :

- le peptide, dit $\alpha171\text{-}185_{Cit}$, ayant la séquence d'acides aminés VDIDIKIX$_1$SCX$_2$GSCS (SEQ ID NO : 8) où X$_1$ et X$_2$ représentent chacun un résidu citrullyl,
- le peptide, dit $\alpha621\text{-}635_{Cit}$, ayant la séquence d'acides aminés X$_1$GHAKSX$_2$PVX$_3$GIHTS (SEQ ID NO : 12) où X$_1$, X$_2$ et X$_3$ représentent chacun un résidu citrullyl,
- le peptide, dit $\beta60\text{-}74_{Cit\text{-}NH2}$, ayant la séquence d'acides aminés X$_1$PAPPPISGGGYX$_2$AX$_3$ (SEQ ID NO: 15) où X$_1$ et X$_2$ représentent chacun un résidu citrullyl et X$_3$ représente un dérivé citrullyl avec un groupement carboxamide à la place du groupement carboxyle et
- le peptide, dit $_{Ac}\text{-}\alpha36\text{-}50_{Cit}$, ayant la séquence d'acides aminés GPX$_1$VVEX$_2$HQSACKDS (SEQ ID NO : 6) où le résidu G en N-terminal est acétylé et où X$_1$ et X$_2$ représentent chacun un résidu citrullyl et/ou le peptide, dit $\alpha36\text{-}50_{Cit}$, ayant la séquence d'acides aminés GPX$_1$VVEX$_2$HQSACKDS (SEQ ID NO : 5) où X$_1$ et X$_2$ représentent chacun un résidu citrullyl.

**8.** Utilisation d'une composition comprenant au moins un peptide citrulliné choisi dans le groupe consistant en les peptides ayant des séquences SEQ ID NO : 8, SEQ ID NO : 12, SEQ ID NO : 15, SEQ ID NO : 6 et SEQ ID NO : 5 dans la fabrication d'une colonne d'aphérèse selon l'une quelconque des revendications 1 à 7.

**9.** Composition comprenant au moins un peptide citrulliné choisi dans le groupe consistant en les peptides ayant des séquences SEQ ID NO : 8, SEQ ID NO : 12, SEQ ID NO : 15, SEQ ID NO : 6 et SEQ ID NO : 5 pour son utilisation dans le traitement ou la prévention d'une maladie auto-immune à auto-anticorps anti-protéine citrullinée où le ou les peptides de la composition sont immobilisés sur un support solide chargé dans une colonne d'aphérèse.

**10.** Composition pour son utilisation selon la revendication 9 **caractérisée en ce que** la maladie auto-immune à auto-anticorps anti-protéines citrullinées est choisie dans le groupe du syndrome de Sjögren, de l'arthrite juvénile idiopathique et de la polyarthrite rhumatoïde, de préférence la polyarthrite rhumatoïde.

**11.** Composition pour son utilisation selon les revendications 9 ou 10 **caractérisée en ce que** le sujet traité a un haut titre en auto-anticorps anti-protéines citrullinées (AAPC).

**Patentansprüche**

**1.** Apherese-Säule, die mit einem festen Träger geladen ist, der eine Zusammensetzung umfasst, die mindestens ein Peptid umfasst, das aus der Gruppe ausgewählt ist, bestehend aus:

- dem sogenannten $\alpha171\text{-}185_{Cit}$ Peptid, das die Aminosäuresequenz VDIDIKIX$_1$SCX$_2$GSCS (SEQ ID NO: 8) aufweist, wobei X$_1$ und X$_2$ jeweils einen Citrullyl-Rückstand darstellen,
- dem sogenannten $\alpha621\text{-}635_{Cit}$ Peptid, das die Aminosäuresequenz X$_1$GHAKSX$_2$PVX$_3$GIHTS (SEQ ID NO: 12)

aufweist, wobei $X_1$, $X_2$ und $X_3$ jeweils einen Citrullyl-Rückstand darstellen,

- dem sogenannten $\beta$60-74$_{Cit-NH2}$ Peptid, das die Aminosäuresequenz $X_1$PAPPPISGGGY$X_2$A$X_3$ (SEQ ID NO: 15) aufweist, wobei $X_1$ und $X_2$ jeweils einen Citrullyl-Rückstand darstellen und $X_3$ ein Citrullyl-Derivat mit einer Carboxamidgruppe anstelle der Carboxylgruppe darstellt, und

- dem sogenannten $_{Ac}$-$\alpha$36-50$_{Cit}$ Peptid, das die Aminosäuresequenz GP$X_1$VVE$X_2$HQSACKDS (SEQ ID NO: 6) aufweist, wobei der G-Rückstand am N-Terminus acetyliert ist und wobei $X_1$ und $X_2$ jeweils einen Citrullyl-Rückstand darstellen und/oder dem sogenannten $\alpha$36-50$_{Cit}$ Peptid, das die Aminosäuresequenz GP$X_1$VVE$X_2$HQSACKDS (SEQ ID NO: 5) aufweist, wobei $X_1$ und $X_2$ jeweils einen Citrullyl-Rückstand darstellen, dessen Peptid oder Peptide direkt oder indirekt auf dem Träger immobilisiert ist (sind).

2. Apherese-Säule nach Anspruch 1, **dadurch gekennzeichnet, dass** sie mindestens 2 Peptide ausgewählt aus der Gruppe bestehend aus $\alpha$36-50$_{Cit}$, $_{Ac}$-$\alpha$36-50$_{Cit}$, $\alpha$171-185$_{Cit}$, $\alpha$621-635$_{Cit}$ und $\beta$60-74$_{Cit-NH2}$ umfasst.

3. Apherese-Säule nach Anspruch 1 oder 2, **dadurch gekennzeichnet, dass** sie die $\alpha$171-185$_{Cit}$, $\alpha$621-635$_{Cit}$, $\beta$60-74$_{Cit-NH2}$ Peptide und das (die) $\alpha$36-50$_{Cit}$ und/oder $_{Ac}$-$\alpha$36-50$_{Cit}$ Peptid(e) umfasst.

4. Apherese-Säule nach einem der Ansprüche 1 bis 3, **dadurch gekennzeichnet, dass** sie weiter das $\alpha$501-515$_{Cit}$ Peptid der Aminosäuresequenz SGIGTLOGF$X_1$H$X_2$HPO (SEQ ID NQ: 10) umfasst, wobei $X_1$ und $X_2$ jeweils einen Citrullyl-Rückstand darstellen.

5. Apherese-Säule nach einem der Ansprüche 1 bis 4, **dadurch gekennzeichnet, dass**:

- das oder die Peptid(e), ausgewählt aus der Gruppe bestehend aus $\alpha$171-185$_{Cit}$, $\alpha$621-635$_{Cit}$, und $\beta$60-74$_{Cit-NH2}$, und optional das Peptid $\alpha$501-515$_{Cit}$, direkt oder indirekt durch ihr N-terminales Ende auf dem festen Träger immobilisiert sind, und/oder
- das (die) $_{Ac}$-$\alpha$36-50$_{Cit}$ und/oder $\alpha$36-50$_{Cit}$ Peptid(e) direkt oder indirekt durch ihr C-terminales Ende auf dem festen Träger immobilisiert sind.

6. Apherese-Säule nach einem der Ansprüche 1 bis 5 **dadurch gekennzeichnet, dass** das oder die Peptid(e) indirekt mittels einer oder mehrerer Zwischenverbindungen auf dem festen Träger immobilisiert ist (sind).

7. Apherese-Säule, die mit einem festen Träger geladen ist, der eine Zusammensetzung umfasst, die mindestens zwei Peptide umfasst, die aus der Gruppe ausgewählt sind, bestehend aus:

- dem sogenannten $\alpha$171-185$_{Cit}$ Peptid, das die Aminosäuresequenz VDIDIKI$X_1$SC$X_2$GSCS (SEQ ID NO: 8) aufweist, wobei $X_1$ und $X_2$ jeweils einen Citrullyl-Rückstand darstellen,
- dem sogenannten $\alpha$621-635$_{Cit}$ Peptid, das die Aminosäuresequenz $X_1$GHAKS$X_2$PV$X_3$GIHTS (SEQ ID NO: 12) aufweist, wobei $X_1$, $X_2$ und $X_3$ jeweils einen Citrullyl-Rückstand darstellen,
- dem sogenannten $\beta$60-74$_{Cit-NH2}$ Peptid, das die Aminosäuresequenz $X_1$PAPPPISGGGY$X_2$A$X_3$ (SEQ ID NO: 15) aufweist, wobei $X_1$ und $X_2$ jeweils einen Citrullyl-Rückstand darstellen und $X_3$ ein Citrullyl-Derivat mit einer Carboxamidgruppe anstelle der Carboxylgruppe darstellt, und
- dem sogenannten $_{Ac}$-$\alpha$36-50$_{Cit}$ Peptid, das die Aminosäuresequenz GP$X_1$VVE$X_2$HQSACKDS (SEQ ID NO: 6) aufweist, wobei der G-Rückstand am N-Terminus acetyliert ist und wobei $X_1$ und $X_2$ jeweils einen Citrullyl-Rückstand darstellen und/oder dem sogenannten $\alpha$36-50$_{Cit}$ Peptid, das die Aminosäuresequenz GP$X_1$VVE$X_2$HQSACKDS (SEQ ID NO: 5) aufweist, wobei Xi und X2 jeweils einen Citrullyl-Rückstand darstellen.

8. Verwendung einer Zusammensetzung, die mindestens ein citrulliniertes Peptid umfasst, das aus der Gruppe bestehend aus den Peptiden ausgewählt ist, die Sequenzen SEQ ID NO: 8, SEQ ID NO: 12, SEQ ID NO: 15, SEQ ID NO: 6 und SEQ ID NO: 5 aufweisen, bei der Herstellung einer Apherese-Säule nach einem der Ansprüche 1 bis 7.

9. Zusammensetzung, die mindestens ein citrulliniertes Peptid umfasst, das aus der Gruppe bestehend aus den Peptiden ausgewählt ist, die Sequenzen SEQ ID NO: 8, SEQ ID NO: 12, SEQ ID NO: 15, SEQ ID NO: 6 und SEQ ID NO: 5 aufweisen zu deren Verwendung bei der Behandlung oder Vorbeugung einer Autoimmmun-Erkrankung mit anti-citrulliniertem Protein-Autoantikörpern, wobei das oder die Peptid(e) der Zusammensetzung auf einem in eine Apherese-Säule geladenen festen Träger immobilisiert ist (sind).

10. Zusammensetzung zu deren Verwendung nach Anspruch 9 **dadurch gekennzeichnet, dass** die Autoimmmun-Erkrankung mit anti-citrulliniertem Protein-Autoantikörpern aus der Gruppe des Sjögren's Syndroms, der juvenilen idiopathischen Arthritis und der rheumatoiden Arthritis, vorzugsweise die rheumatoide Arthritis, ausgewählt ist.

**11.** Zusammensetzung zu deren Verwendung nach einem der Ansprüche 9 oder 10, **dadurch gekennzeichnet, dass** das behandelte Subjekt hohe anti-citrullinierte Protein-Autoantikörperwerte (ACPA) aufweist.

**Claims**

**1.** Apheresis column loaded with a solid support comprising a composition that includes at least one peptide selected from the group consisting of:

- the peptide, called $\alpha 171\text{-}185_{Cit}$, having the amino acid sequence VDIDIKIX$_1$SCX$_2$GSCS (SEQ ID NO: 8) where X$_1$ and X$_2$ each represent a citrullyl residue,
- the peptide, called $\alpha 621\text{-}635_{Cit}$, having the amino acid sequence X$_1$GHAKSX$_2$PVX$_3$GIHTS (SEQ ID NO: 12) where X$_1$, X$_2$ and X$_3$ each represent a citrullyl residue,
- the peptide, called $\beta 60\text{-}74_{Cit\text{-}NH2}$, having the amino acid sequence X$_1$PAPPPISGGGYX$_2$AX$_3$ (SEQ ID NO: 15) wherein X$_1$ and X$_2$ each represent a citrullyl residue and X$_3$ represents a citrullyl derivative with a carboxamide group in place of the carboxyl group, and
- the peptide, called $_{Ac}\text{-}\alpha 36\text{-}50_{Cit}$, having the amino acid sequence GPX$_1$WEX$_2$HQSACKDS (SEQ ID NO: 6) wherein the G residue at the N-terminal is acetylated and wherein X$_1$ and X$_2$ each represent a citrullyl residue and/or the peptide, referred to as $\alpha 36\text{-}50_{Cit}$, having the amino acid sequence GPX$_1$VVEX$_2$HQSACKDS (SEQ ID NO: 5) wherein X$_1$ and X$_2$ each represent a citrullyl residue wherein the peptide(s) are immobilized directly or indirectly on the support.

**2.** Apheresis column according to claim 1, **characterized in that** it comprises at least 2 peptides selected from the group consisting of $\alpha 36\text{-}50_{Cit}$, $_{Ac}\text{-}\alpha 36\text{-}50_{Cit}$, $\alpha 171\text{-}185_{Cit}$, $\alpha 621\text{-}635_{Cit}$ and $\beta 60\text{-}74_{Cit\text{-}NH2}$.

**3.** Apheresis column according to claim 1 or 2, **characterized in that** it comprises the peptides $\alpha 171\text{-}185_{Cit}$, $\alpha 621\text{-}635_{Cit}$, $\beta 60\text{-}74_{Cit\text{-}NH2}$ and the peptide(s) $\alpha 36\text{-}50_{Cit}$ and/or $_{Ac}\text{-}\alpha 36\text{-}50_{Cit}$.

**4.** Apheresis column according to any one of claims 1 to 3, **characterized in that** it further comprises the peptide $\alpha 501\text{-}515_{Cit}$ with the amino acid sequence SGIGTLDGFX$_1$HX$_2$HPD (SEQ ID NO: 10) wherein X$_1$ and X$_2$ each represent a citrullyl residue.

**5.** Apheresis column according to any one of claims 1 to 4, **characterized in that**:

- the peptide(s) selected from the group consisting of $\alpha 171\text{-}185_{Cit}$, $\alpha 621\text{-}635_{Cit}$, and $\beta 60\text{-}74_{Cit\text{-}NH2}$, and optionally the peptide $\alpha 501\text{-}515_{Cit}$, are immobilized directly or indirectly on the solid support by their N-terminal end and/or
- the peptide(s) $_{Ac}\text{-}\alpha 36\text{-}50_{Cit}$ and/or $\alpha 36\text{-}50_{Cit}$ are immobilized directly or indirectly on the solid support by their C-terminal end.

**6.** Apheresis column according to any of claims 1 to 5, **characterized in that** the peptide(s) are indirectly immobilized on the solid support by means of one or more intermediate compounds.

**7.** Apheresis column loaded with a solid support comprising a composition that includes at least two peptides selected from the group consisting of:

- the peptide, called $\alpha 71\text{-}185_{Cit}$, having the amino acid sequence VDIDIKIX$_1$SCX$_2$GSCS (SEQ ID NO: 8) where X$_1$ and X$_2$ each represent a citrullyl residue,
- the peptide, called $\alpha 621\text{-}635_{Cit}$, having the amino acid sequence X$_1$GHAKSX$_2$PVX$_3$GIHTS (SEQ ID NO: 12) where X$_1$, X$_2$ and X$_3$ each represent a citrullyl residue,
- the peptide, called $\beta 60\text{-}74_{Cit\text{-}NH2}$, having the amino acid sequence X$_1$PAPPPISGGGYX$_2$AX$_3$ (SEQ ID NO: 15) wherein X$_1$ and X$_2$ each represent a citrullyl residue and X$_3$ represents a citrullyl derivative with a carboxamide group in place of the carboxyl group, and
- the peptide, called $_{Ac}\text{-}\alpha 36\text{-}50_{Cit}$, having the amino acid sequence GPX$_1$VVEX$_2$HQSACKDS (SEQ ID NO: 6) wherein the G residue at the N-terminal is acetylated and wherein X$_1$ and X$_2$ each represent a citrullyl residue and/or the peptide, referred to as $\alpha 36\text{-}50_{Cit}$, having the amino acid sequence GPX$_1$VVEX$_2$HQSACKDS (SEQ ID

NO: 5) wherein $X_1$ and $X_2$ each represent a citrullyl residue.

8. Use of a composition comprising at least one citrullinated peptide selected from the group consisting of peptides having sequences SEQ ID NO: 8, SEQ ID NO: 12, SEQ ID NO: 15, SEQ ID NO: 6, and SEQ ID NO: 5 in the manufacture of an apheresis column according to any one of claims 1 to 7.

9. Composition comprising at least one citrullinated peptide selected from the group consisting of peptides having sequences SEQ ID NO: 8, SEQ ID NO: 12, SEQ ID NO:15, SEQ ID NO: 6, and SEQ ID NO: 5 for use in the treatment or prevention of an autoimmune disease with anti-citrullinated protein autoantibodies, wherein the peptide(s) of the composition are immobilized on a solid support loaded into an apheresis column.

10. Composition for use according to claim 9, **characterized in that** the autoimmune disease with anti-citrullinated protein autoantibodies is selected from the group consisting of Sjögren's syndrome, juvenile idiopathic arthritis, and rheumatoid arthritis, preferably rheumatoid arthritis.

11. Composition for use according to claims 9 or 10, **characterized in that** the treated subject has a high titer of anti-citrullinated protein autoantibodies (ACPA).

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2908134

**Littérature non-brevet citée dans la description**

- **RANTAPÄÄ-DAHLQVIST S et al.** *Arthritis Rheum.*, October 2003, vol. 48 (10), 2741-9
- **NIELEN MM et al.** *Arthritis Rheum.*, February 2004, vol. 50 (2), 380-6
- **DE RYCKE L et al.** *Ann. Rheum. Dis.*, 2004, vol. 63 (12), 1587-1593
- **L. ROSTAING et al.** Treatment of large plasma volumes using specific immunoadsorption to desensitize ABO-incompatible kidney transplant candidates. *J. Nephropathology.*, 2016, vol. 5 (3), 90-97